# EUROPEAN PATENT APPLICATION

(11) **EP 4 647 026 A1**
(43) Date of publication of application: **12.11.2025**
(21) Application number: 24174349.1
(22) Date of filing: 06.05.2024
(51) Int. Cl.: A61B 18/20

(54) **LIGHT-BASED SKIN TREATMENT APPARATUS, SENSOR SYSTEM, AND METHOD FOR OPERATING THE APPARATUS**

(71) Applicant: Koninklijke Philips N.V., 5656 AG Eindhoven (NL)
(72) Inventor: BOAMFA, Marius Iosif, Eindhoven (NL); BOURQUIN, Yannyk Parulian Julian, Eindhoven (NL); NUIJS, Antonius Maarten, 5656AG Eindhoven (NL)
(74) Representative: Philips Intellectual Property & Standards

(57) **Abstract**

There is provided light-based skin treatment apparatus, comprising a treatment light source adapted to provide treatment light to a skin, and a sensor system for measuring a characteristic of the skin. The sensor system comprises a detection unit having a detector and at least one sensor light source. The at least one sensor light source is adapted to emit sensor light to the skin. The detector is adapted to generate a detector signal in response to receiving at least a portion of the sensor light after interaction of the sensor light with the skin. The sensor system comprises a heat control system adapted to control a temperature of the detection unit at a desired temperature above an ambient temperature.

## Description

### FIELD OF THE INVENTION

The invention relates to light-based skin treatment apparatus, such as an intense pulsed light (IPL) apparatus. The invention further relates to a sensor system for use in a light-based skin treatment apparatus. The invention further relates to a method for operating a light-based skin treatment apparatus.

### BACKGROUND OF THE INVENTION

Intense Pulsed Light (IPL) technology is a popular solution for many applications, such as photo-epilation, lesion treatment, photo-rejuvenation, in-home personal care, professional personal care, and medical settings. For photo-epilation in a home environment, IPL photo-epilation apparatus applies broad-spectrum light to the surface of the skin, targeting the melanin in the hair and hair follicles. Hairs and follicles that are in their anagen phase of the growth cycle absorb the light's energy and go into a resting phase. This prevents the re-growth of hair. For effective use of IPL technology for hair removal, such as minimize damage, thermal injury/burns or irritation to the skin, the energy setting of the IPL photo-epilation apparatus can be adapted based on the skin tone of the skin. Some IPL photo-epilation apparatus, e.g. the Philips Lumea Prestige, can detect the skin tone before and during a treatment, and select an appropriate energy setting. The skin tone can be detected and categorized into one of, e.g. six, different types. The skin types can be broadly labelled as: 'white', 'beige', 'light brown', 'medium brown', 'dark brown' and 'darker'. Typically, IPL photo-epilation apparatus should not be used with the darker skin type as the skin rather than the hair or follicle will absorb the light's energy. If a skin type labelled as 'darker' is detected, the apparatus does not trigger a flash.

A method of skin type detection in IPL apparatus uses reflectance spectroscopy, for example as described in "A portable reflectometer for a rapid quantification of cutaneous haemoglobin and melanin" by Feather J.W., Ellis D. J., and Leslie G., Phys. Med. Biol. 1988, 33(6), 711 -722. In this technique, the ratio between two reflected wavelengths is used to compute a melanin index. The melanin index is then used to compute skin type. The two wavelengths are red and near infrared. Melanin has a higher absorption coefficient at these two wavelengths compared to water and haemoglobin. One type of skin tone sensor that is currently used in IPL apparatus makes use of two light emitting diodes (LEDs) that operate at the two distinct wavelengths. The two wavelengths are 640 nm and 870 nm central wavelengths respectively. The light of these two LEDs is emitted towards the skin and a detector measures the reflected light, resulting in detector signals for the two LEDs respectively. Based on the levels of skin reflectivity for the two wavelengths, a skin tone can be computed. The skin tone is a function of the ratio between the two reflected wavelengths.

### SUMMARY OF THE INVENTION

However, the wavelengths of the light emitted by the LEDs are dependent on the temperature of the LEDs. During use of the IPL apparatus, the temperature of the LEDs and the detector increases. The change in the temperature of the LEDs may cause a difference in the ratio between the two reflected wavelengths. Further, for a certain wavelength of light received by the detector, the detector signal may differ depending on the temperature of the detector. As a result, the skin tone is determined with limited accuracy. The limited accuracy may lead to mischaracterization of the skin. The same problem of mischaracterization of the skin is present also in other types of light-based skin treatment apparatus that use laser light or LED light instead of IPL light. The heat generated by the light source, e.g., the laser or LED, that generates the treatment light causes the temperature to increase.

It is an objective of the invention to improve a characterization of the skin to improve the control of a light-based skin treatment apparatus.

According to a first aspect, there is provided a light-based skin treatment apparatus, comprising an treatment light source adapted to provide treatment light to a skin, and a sensor system for measuring a characteristic of the skin. The sensor system comprises a detection unit having a detector and at least one sensor light source. The at least one sensor light source is adapted to emit sensor light to the skin. The detector is adapted to generate a detector signal in response to receiving at least a portion of the sensor light after interaction of the sensor light with the skin. The sensor system comprises a heat control system adapted to control a temperature of the detection unit at a desired temperature above an ambient temperature.

During use of the light-based skin treatment apparatus, heat is generated, for example by an IPL light source, by a laser light source, or by an LED light source. Some of this heat is transferred to the detection unit. The heat control system controls the temperature of the detection unit at the desired temperature by providing additional heat to the detection unit. In case only a small amount of heat is transferred from the treatment light source to the detection unit, the heat control system provides a large amount of heat to maintain the detection unit at the desired temperature. In case a large amount of heat is transferred from the treatment light source to the detection unit, the heat control system provides only a small amount of heat or no heat at all to maintain the detection unit at the desired temperature. This way, the detection unit is kept at the desired temperature in an energy efficient way. Heating the detection unit in this way is more energy-efficient and more cost-effective than cooling the detection unit to remain at the ambient temperature, because heat from the treatment light source is used to control the detection unit at the desired temperature. Because the detection unit is kept at the desired temperature, the detector signal represents the characteristic of the skin in a more accurate way. With the improved accuracy of the detector signal, an improved characterization of the skin is obtained, allowing for improved control of the light-based skin treatment apparatus.

The treatment light source is adapted to provide light pulses. For example, the treatment light source is adapted to generate light at a high intensity for a short duration, such as for less than 100 ms or less than 50 ms or less than 10 ms or less than 8 ms. The intensity of the light pulse is high enough to perform an operation on the skin. Such operation is, for example, hair removal or photo-rejuvenation or vein treatment or acne treatment. For example, the treatment light source comprises one or more optical filters. For example, the treatment light source comprises an optical filter to prevent light with a potentially damaging wavelength, such as UV light, from being transmitted to the skin. For example, the treatment light source comprises a Xenon flash lamp, or a laser, or multiple lasers, a LED, or multiple LEDs. For example, the treatment light source is adapted to provide treatment light with wavelengths in the range of 530-1200 nanometers. For example, the light-based skin treatment apparatus is an intense pulsed light (IPL) apparatus.

The characteristic of the skin includes, for example, a skin tone, or a skin melanin index, or any other skin parameter relating to skin color. The characteristic of the skin includes, for example, the presence of hair, moles, scars, and/or acne.

The detector is, for example, a photodetector. The detector comprises, for example, a photodiode or multiple photodiodes. For example, the detector comprises a phototransistor or multiple photo-transistors. For example, the detector comprises a charged-coupled device, CCD. For example, the detector comprises a CMOS image sensor. For example, in case the detection unit comprises multiple sensor light sources, the detector comprises a single detector that generates a single detector signal based on light received from all of the multiple sensor light sources. For example, in case the detection unit comprises multiple sensor light sources, the detector comprises multiple detectors, wherein each of the multiple detectors forms a pair with one of the multiple sensor light sources. For each pair, the detector generates the detector signal based only on light received from the one paired sensor light source.

The sensor light source is, for example, a Light-Emitting Diode, LED. For example, the at least one sensor light source comprises two LEDs. The two LEDs operate at two distinct wavelengths. One of the two LEDs is adapted to emit sensor light with a wavelength of 640 nanometers. The other of the two LEDs is adapted to emit sensor light with a wavelength of 870 nanometers. Melanin has a higher absorption coefficient at these two wavelengths compared to water and haemoglobin. Therefore, these two wavelengths are especially suited for measuring a characteristic of the skin. For example, the detector generates the detector signal based on a ratio between these two wavelengths as received by the detector. For example, the sensor light source is a single light source adapted to generate sensor light at the two wavelengths. For example, the at least one sensor light source comprises at least one laser source. The laser source is adapted to generate the sensor light.

The sensor light source is adapted to emit the sensor light to the skin. For example, the sensor light is directly emitted from the sensor light source to the skin. In another example, the sensor light is emitted from the sensor light source via one or more optical components to the skin. For example, the one or more optical components comprise a window or a mirror or a lens or a diffusor.

When the sensor light is incident on the skin, the sensor light interacts with the skin. For example, part of the sensor light is absorbed by the skin, whereas another part of the sensor light is reflected from the skin to the detector. For example, the sensor light reflected from the skin to the detector has less wavelengths or other wavelengths than the sensor light as emitted from the at least one sensor light source. For example, the sensor light reflected from the skin to the detector has less intensity in total or less intensity for one or more wavelengths than the sensor light as emitted from the at least one sensor light source.

The heat control system is adapted to control the temperature of the detection unit at the desired temperature. The desired temperature is above the ambient temperature. For example, when the light-based skin treatment apparatus is turned on by a user, the light-based skin treatment apparatus, including the detection unit, is at an ambient temperature. For example, the ambient temperature is in the range of 18-25°C. The heat control system provides heat to the detection unit to raise the temperature of the detection unit above the ambient temperature till the detection unit reaches the desired temperature. For example, when the detection unit is at the desired temperature, the heat control system stops providing heat to the detection unit. The heat control system starts providing heat again to the detection unit in case the temperature of the detection unit has decreased below a threshold temperature below the desired temperature. In another example, the heat control system provides heat to the detection unit continuously. When the detection unit has reached the desired temperature, there may remain a heat flow from the detection unit to the ambient environment, because the detection unit has a higher temperature than the ambient temperature. The heat control system provides heat to the detection unit continuously to compensate for the heat lost to the ambient environment. For example, the heat control system is adapted to provide a large electric current through the at least one sensor light source. This causes the at least one sensor light source to emit sensor light. However, because of a limited efficiency of the at least one sensor light source, some of the electric energy provided by the large electric current is not converted to the sensor light, but to heat instead. This heat causes the temperature of the detection unit to reach the desired temperature. Preferably, the large electric current is as large as possible to allow the detection unit to reach the desired temperature as fast as possible. However, the large electric current should be low enough to prevent damage to the at least one sensor light source.

For example, the desired temperature is a temperature that is high enough that during use of the light-based skin treatment apparatus, the detection unit does not exceed the desired temperature. This way, the heat control system is able to maintain the detection unit at the desired temperature by adding heat to the detection unit. For example, the light-based skin treatment apparatus is adapted to prevent the treatment light source from heating the detection unit to the desired temperature. For example, the light-based skin treatment apparatus is designed to transfer heat to the ambient environment to prevent the detection unit from exceeding a maximum operating temperature. For example, the light-based skin treatment apparatus has thermal protection that restricts the use of the light-based skin treatment apparatus and/or prevents use of the light-based skin treatment apparatus in case the temperature of the detection unit becomes too high. In another example, the temperature of the detection unit exceeds the desired temperature after a certain time of use of the light-based skin treatment apparatus. In this example, there is a benefit that the sensor system provides an improved measure of the characteristic of the skin when starting with the light-based skin treatment apparatus. This way, the characteristic, such as skin tone, can be accurately determined at the start of using the light-based skin treatment apparatus. The information of this characteristic can then be used for the rest of the usage of the light-based skin treatment apparatus. Alternatively or in addition, when the detector unit is optimized for use at the desired temperature, the detector unit is likely to perform better for a temperature higher than the desired temperature compared to the case that the detector unit is optimized for use at the ambient temperature.

For example, the desired temperature is in the range of 40 - 200°C. The ambient temperature is not likely to be more than 40°C. This allows the heat control system to control the detection unit at the desired temperature by adding heat to the detection unit. A temperature of more than 200°C may cause damage to components of the detection unit. For example, the soldering of the components in the detection unit may not function properly at a temperature of more than 200°C. For example, the desired temperature is in a range of 50-150°C, for example, in a range of 60-90°C.

In an embodiment, the heat control system comprises a heating device. The heating device is adapted to provide heat to the detection unit.

According to this embodiment, the heat control system is adapted to control the temperature of the detection unit at the desired temperature by using the heating device. This way, the temperature of the detection unit is controlled in an efficient way. For example, the heating device comprises a resistor. The heat control system is adapted to provide an electric current through the heating device. The resistor converts the electric current into heat. For example, the heating device comprises a resistance wire, or a resistance ribbon, or a resistance coil. For example, the heating device comprises a thin-film heating element. For example, the heating device comprises a radiative element adapted to generate infrared radiation.

In an embodiment, the detection unit comprises a thermally conductive body. The thermally conductive body comprises a thermally conductive material. The light source and the detector are coupled to the thermally conductive body. The sensor light source, the detector, and the thermally conductive material are thermally coupled to each other.

According to this embodiment, the sensor light source, the detector, and the thermally conductive material are all thermally coupled to each other. As a result, the heat control system is able to control both the sensor light source and the detector at the desired temperature. Also, the heat control system is able to bring both the sensor light source and the detector to the desired temperature in a short amount of time because the heat from the heat control system is evenly divided between the sensor light source and the detector. The thermally conductive material has good properties in view of thermal conductivity. For example, the thermally conductive material has a higher thermal conductivity than other parts of the IPL apparatus, such as the sensor light source, or the detector, or a housing of the IPL apparatus. For example, the thermally conductive material has a thermal conductivity of at least 50 W/mK or more, such as at least 90 W/mK, or at least 100 W/mK, or at least 200 W/mK.

In an embodiment, the heat control system comprises a heating device thermally coupled to the thermally conductive material. The heating device is adapted to provide heat to the thermally conductive material.

According to this embodiment, the heat control system is adapted to control the temperature of the detection unit at the desired temperature by using the heating device. The heat from the heating device is transferred via the thermally conductive material to the at least one sensor light source and the detector. This way, the detection unit reaches the desired temperature in an efficient way. For example, the heating device comprises a resistor. The heat control system is adapted to provide an electric current through the heating device. The resistor converts the electric current into heat. For example, the heating device comprises a resistance wire, or a resistance ribbon, or a resistance coil. For example, the heating device comprises a thin-film heating element. For example, the heating device comprises a radiative element adapted to generate infrared radiation. For example, the heating device is mounted to the thermally conductive body. In another example, the heating device is mounted on to a different part of the light-based skin treatment apparatus than the thermally conductive body, such as the housing of the light-based skin treatment apparatus.

In an embodiment, the thermally conductive body comprises one of a metal core printed circuit board and a ceramic printed circuit board.

According to this embodiment, using a printed circuit board, PCB, allows for easy mounting of the detector and the at least one sensor light source. The PCB also allows for electrical connections to the detector and the at least one sensor light source in an easy way. By using a metal core PCB or a ceramic PCB, the good thermal conductive properties of the metal core PCB and the ceramic PCB are used to efficiently transfer heat to the detector and the at least one sensor light source. In known applications, metal core PCBs and ceramic PCBs are used to provide a heat sink to transfer heat away from components mounted on the PCB. The inventors have discovered that the good thermal conductive properties of the metal core PCB and the ceramic PCB may be used in an opposite way to provide heat to the detector and the at least one sensor light source mounted on the PCB.

In an embodiment, the thermally conductive material comprises aluminum oxide, or aluminum nitride, or boron nitride, or beryllium oxide, or silicon carbide.

These materials provide a good thermal conductivity, and are electrically insulating. This allows the at least one sensor light source and the detector to be thermally coupled to the thermally conductive material without the need for any electrical insulation in between.

In an embodiment, wherein the heat control system comprises a temperature sensor. The temperature sensor is thermally coupled to the thermally conductive material. The temperature sensor is adapted to generate a temperature signal representative of a temperature of the thermally conductive material. The heat control system is configured to control the temperature of the detection unit based on the temperature signal.

According to this embodiment, the heat control system controls the temperature of the detection unit based on the temperature signal. The temperature signal is representative of the temperature of the thermally conductive material. Because the thermally conductive material, the detector, and the at least one sensor light source are thermally coupled to each other, the temperature of the detection unit is controlled in an effective way based on the temperature signal.

In an embodiment, the heat control system is configured to control the temperature of the detection unit at a first desired temperature in case the temperature of the thermally conductive material is lower than a threshold temperature. The control system is configured to control the temperature of the detection unit at a second desired temperature in case the temperature of the thermally conductive material is higher than a threshold temperature. The second desired temperature is higher than the first desired temperature.

According to this embodiment, in case the thermally conductive material is at a temperature lower than the threshold temperature, the heat control system increases the temperature of the thermally conductive material till the temperature reaches the first desired temperature. During use of the light-based skin treatment apparatus, additional heat from the treatment light source is transferred to the detection unit. Because of this additional heat, the temperature of the thermally conductive material may exceed the first desired temperature. For example, the threshold temperature is higher than the desired temperature. If the temperature of the thermally conductive material exceeds the threshold temperature, the heat control system further heats the thermally conductive material till the temperature reaches the second desired temperature. The sensor system is, for example, optimized for both the first desired temperature and the second desired temperature. For example, the heat control system is adapted to generate a signal indicative of whether the detection unit is at the first desired temperature or the second desired temperature. For example, the detector is adapted to generate the detector signal based on the signal indicative of whether the detection unit is at the first desired temperature or the second desired temperature. By having the first desired temperature, the heat control system only has to provide a small amount of heat to the thermally conductive body when a user starts using the light-based skin treatment apparatus. This allows the detection unit to reach the first desired temperature quickly and in an energy efficient way. If use of the light-based skin treatment apparatus continues, the heat from the treatment light source may cause the detection unit to exceed the first desired temperature. If that happens, the heat control system is able to set the temperature of the thermally conductive body to the second desired temperature. This improves the accuracy of the sensor system during use of the light-based skin treatment apparatus.

For example, the first desired temperature is 30°C, or 35°C, or 40°C, or 45°C. For example, the second desired temperature is in the range of 50-150°C, for example, in a range of 60-90°C. For example, the threshold temperature is 0.5°C, or 1°C, or 2°C or 5°C higher than the first desired temperature. In an example, the first desired temperature is 30°C, the threshold temperature is 31°C, and the second desired temperature is 65°C. When starting the use of the IPL apparatus, the thermally conductive material is at ambient temperature. In response, the heat control system heats the thermally conductive material till the thermally conductive material is at the first desired temperature of 30°C. After some time of use of the light-based skin treatment apparatus, heat from the treatment light source causes the thermally conductive material to reach the threshold temperature of 31°C. When this occurs, the heat control system further increases the temperature of the thermally conductive material till the thermally conductive material reaches the second desired temperature of 65°C. During further use of the light-based skin treatment apparatus, the temperature of the thermally conductive material does not exceed the second desired temperature of 65°C.

In an embodiment, the light-based skin treatment apparatus comprises a light control system adapted to provide a light control signal to the treatment light source to control operation of the treatment light source. The heat control system is configured to control the temperature of the detection unit based on the light control signal.

According to this embodiment, the light control system controls the operation of the treatment light source by providing the light control signal. The light control signal is representative of the operation of the treatment light source. The treatment light source can generate a large amount of heat depending on the operation of the treatment light source. The heat control system makes use of the light control signal to estimate the amount of heat generated by the treatment light source. For example, if the light control signal is representative of an operation of the treatment light source that generates a small amount of heat, the heat control system provides a large amount of heat to the detection unit to achieve the desired temperature. For example, if the light control signal is representative of an operation of the treatment light source that generates a large amount of heat, the heat control system provides a small amount of heat to the detection unit to achieve the desired temperature. The heat control system takes, for example, a time constant into account. The time constant represents the time it takes for the heat generated by the treatment light source to increase the temperature of the detection unit. For example, the heat control system is adapted to make use of a thermal model that includes the time constant. The thermal model provides a correlation between the light control signal and the heat flow from the treatment light source received by the detection unit.

In an embodiment, the operation of the treatment light source comprises an operation time and/or a flashing frequency and/or an optical fluence of the treatment light source.

According to this embodiment, the operation of the treatment light source comprises the operation time, or the flashing frequency, or the optical fluence of the treatment light source, or any combination of the operation time, the flashing frequency, and the optical fluence of the treatment light source. The operation time is, for example, representative for the time the light-based skin treatment apparatus is in an on-modus. For example, the operation time starts when the user switches the light-based skin treatment apparatus on. For example, the operation starts when the user uses the light-based skin treatment apparatus to generate the first pulse of treatment light. The operation time is indicative of the amount of heat generated by the treatment light source. The flashing frequency is, for example, representative of the number of pulses of treatment light generated by the treatment light source per second or per minute. The flashing frequency is, for example, representative of average number of pulses of treatment light generated by the treatment light source per second or per minute over the last one minute or five minutes or 10 minutes. The flashing frequency is, for example, representative of the number of pulses of treatment light generated by the treatment light source divided by the operation time. The optical fluence is the radiant energy of the treatment light received by the skin per surface area of the skin. The maximum optical fluence of the light-based skin treatment apparatus is, for example 10 J/cm², or for example 6.5 J/cm². Depending on the characteristic of the skin as represented by the detector signal, or by an operation by the user of the treatment apparatus, the optical fluence may be set to a lower value than the maximum value. For example, for some skin types a low optical fluence is sufficient to obtain the desired effect, such as hair removal. Some users may experience skin irritation when using the light-based skin treatment apparatus at a high optical fluence, and may therefore set the light-based skin treatment apparatus to a lower optical fluence. The amount of heat generated by the treatment light source depends on the amount of optical fluence generated by the treatment light source.

In an embodiment, the detector is adapted to generate the detector signal based on sensor light source information. The sensor light source information comprises information about a spectral output of the at least one sensor light source at the desired temperature.

According to this embodiment, the light source information is used to generate the detector signal. For example, information about a spectral output of the at least one sensor light source at the desired temperature is based on a calibration in which the at least one light source is operated at the desired temperature, and the spectral output is measured. For example, the sensor light source information comprises information about an intensity of the sensor light source, or a spectrum of the sensor light source. By using the light source information, one or more properties of the sensor light as emitted by the sensor light source are known. In case there are any differences in these properties in the sensor light as received by the detector, the detector is able to determine the characteristic of the skin with improved accuracy based on these differences. For example, information about a spectral output of the at least one sensor light source at the desired temperature is based on information provided by a manufacturer of the sensor light source.

In an embodiment, the detector is adapted to generate the detector signal based on detector information. The detector information comprises information about a correlation between sensor light received by the detector and the detector signal generated in response to the sensor light received by the detector at the desired temperature.

According to this embodiment, the detector information is used to generate the detector signal. For example, information about a correlation between sensor light received by the detector and the detector signal generated in response to the sensor light received by the detector at the desired temperature is based on a calibration in which the detector is at the desired temperature, and is irradiated with sensor light with one or more known properties. For example, the one or more known properties include spectrum and/or intensity and/or wavelength. By using the detector information, one or more properties of the detector in response to sensor light received by the detector are known. As a result, the detector is able to determine the characteristic of the skin with improved accuracy. For example, information about the correlation between sensor light received by the detector and the detector signal generated in response to the sensor light received by the detector at the desired temperature is based on information provided by a manufacturer of the detector.

According to a second aspect of the invention, there is provided a sensor system for measuring a characteristic of the skin. The sensor system is adapted for use in the light-based skin treatment apparatus according to the first aspect of the invention.

According to a third aspect of the invention, there is provided a method for operating light-based skin treatment apparatus. The method comprises providing, with a treatment light source, IPL light to a skin. The method comprises emitting, with a light source, light to the skin. The method comprises detecting, with a detector, at least a portion of the light after interaction of the light with the skin. The method comprises controlling a temperature of the light source and/or the detector at a desired temperature above an ambient temperature. For example, the light-based skin treatment apparatus is an IPL apparatus.

In an embodiment, the method comprises receiving a temperature signal representative of the temperature of the light source and/or the detector. The method comprises controlling the temperature of the light source and/or the detector at a first desired temperature in case the temperature of the light source and/or the detector is lower than a threshold temperature. The method comprises controlling the temperature of the light source and/or the detector at a second desired temperature in case the temperature of the light source and/or the detector is higher than the threshold temperature. The second desired temperature is higher than the first desired temperature.

### BRIEF DESCRIPTION OF THE DRAWINGS

Exemplary embodiments will now be described, by way of example only, with reference to the following Figures, in which:
FIG. 1 depicts a light-based skin treatment apparatus according to a first embodiment of the invention;
FIGs 2 and 3 depict a sensor system of the light-based skin treatment apparatus according to the first embodiment;
FIG. 4 depicts sensor light source information of LED 1 and LED2;
FIG. 5 depicts a thermally conductive body according to the first embodiment;
FIG. 6 depicts a thermally conductive body according to a second embodiment;
FIG. 7 depicts a sensor system according to a third embodiment;
FIG. 8 depicts a temperature of the detection unit according to the third embodiment;
FIG. 9 depicts a temperature of the detection unit according to a fourth embodiment;
FIG. 10 depicts a light-based skin treatment apparatus according to a fifth embodiment;
FIG. 11 depicts a method for operating the light-based skin treatment apparatus according to a sixth embodiment.

### DETAILED DESCRIPTION OF EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

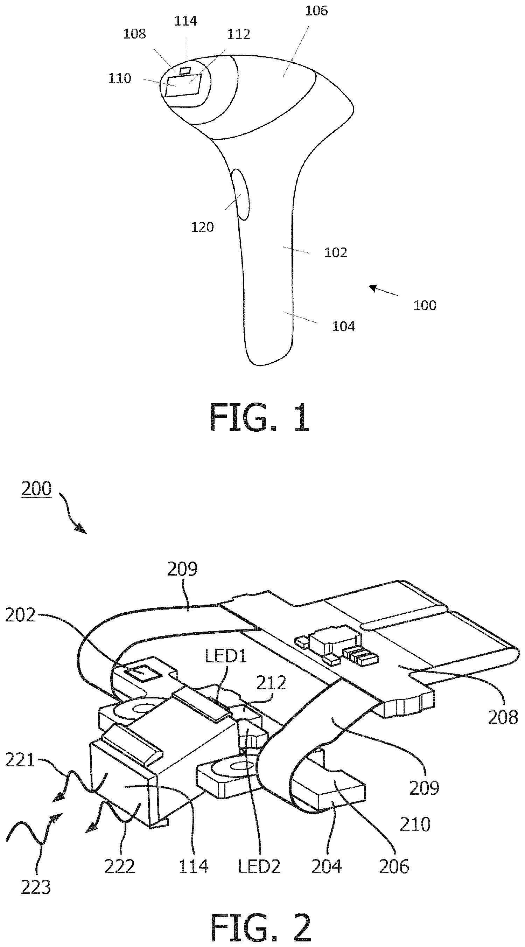

FIG. 1 depicts a light-based skin treatment apparatus, in this case an Intense Pulsed Light, IPL, apparatus 100 according to a first embodiment of the invention. The IPL apparatus 100 is adapted for use on the skin of a subject, e.g. a person or an animal. The IPL apparatus 100 comprises a housing 102 that has a handle portion 104 and a head portion 106. The handle portion 104 is shaped to enable the user to hold the IPL apparatus 100. The head portion 106 has a head end 108 that is to be placed into contact with the subject in order for the IPL apparatus 100 to perform a treatment operation on the skin of the subject.

The IPL apparatus 100 comprises a treatment light source adapted to provide treatment light to the skin. The treatment light source is arranged inside the head portion 106. The IPL apparatus 100 performs the treatment operation by providing pulses of the treatment light to the skin via the aperture 110. The aperture 110 is arranged in or on the housing 102 so that the aperture 110 can be placed adjacent to or on (i.e. in contact with) the skin of the subject. An IPL window 112 is arranged in the aperture 110. The intensity of the light pulses from the treatment light source are high enough to perform the treatment operation on the skin or body part adjacent to the aperture 110.

The IPL apparatus 100 comprises a user control 120 adapted to be operated by the user to activate the IPL apparatus 100. When the user activates the IPL apparatus 100 via the user control 120, the IPL apparatus 100 is able to perform the treatment operation. The user control 120 may be in the form of a switch, a button, a touch pad, etc.

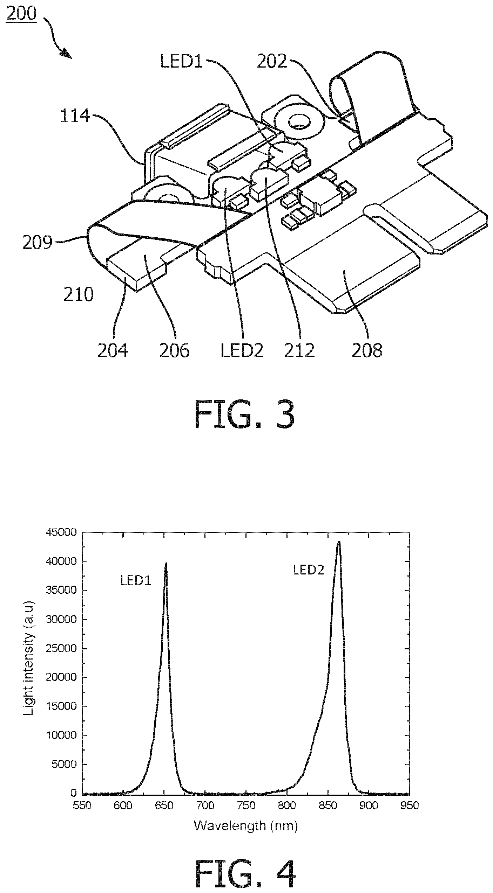

The IPL apparatus 100 comprises a sensor system 200 for measuring a characteristic of the skin. The sensor system 200 is further depicted in FIGs 2 and 3.

FIGs 2 and 3 depict a sensor system 200 of the light-based skin treatment apparatus, in this case the IPL apparatus 100, according to the first embodiment. The sensor system 200 is for measuring a characteristic of the skin. The sensor system 200 is adapted for use in the IPL apparatus 100.

The sensor system 200 comprises a detection unit 210 having a detector 212 and at least one sensor light source. In this embodiment, there are two sensor light sources, i.e., LED1 and LED2. Each of LED 1 and LED2 are adapted to emit sensor light to the skin. LED2 is adapted to generate sensor light 221 with a first wavelength. LED2 is adapted to generate sensor light 222 with a second wavelength different from the first wavelength. For example, the first wavelength is 640 nm, and the second wavelength is 870 nm. The detector 212 is adapted to generate a detector signal in response to receiving at least a portion 223 of the sensor light 221, 222 after interaction of the sensor light 221, 222 with the skin. The sensor system 200 comprises a heat control system adapted to control a temperature of the detection unit 210 at a desired temperature above an ambient temperature.

The sensor light 221, 222 emitted by LED1 and LED2 propagates via a sensor window 114 to the skin. After interaction with the skin, at least a portion 223 of the sensor light propagates back via the sensor window 114 to the detector 212.

The heat control system comprises a heating device 202. The heating device 202 is adapted to provide heat to the detection unit 210.

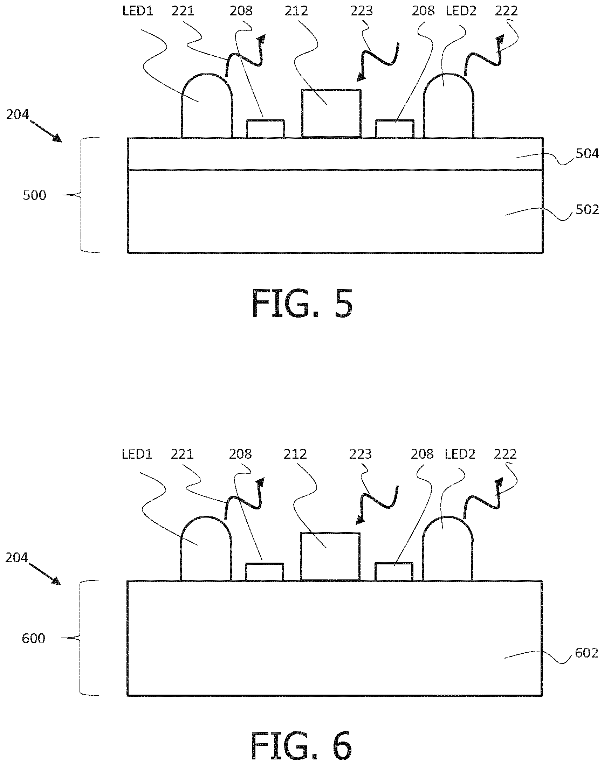

The detection unit 210 comprises a thermally conductive body 204. The thermally conductive body 204 comprises a thermally conductive material 206, as for example shown in FIGs 5 and 6. The sensor light source LED1, LED2, and the detector 212 are coupled to the thermally conductive body 204. In this embodiment, the sensor light source LED1, LED2, and the detector 212 are mounted on the thermally conductive body 204. The sensor light source, the detector 212, and the thermally conductive material 206 are thermally coupled to each other.

The heating device 202 is thermally coupled to the thermally conductive material 206. The heating device 202 is adapted to provide heat to the thermally conductive material 206.

The detector 212, LED1 and LED 2 are connected via electric traces 209 to an electric connector 208.

FIG. 4 depicts sensor light source information of LED 1 and LED2. The light source information of LED 1 and LED 2 comprises the light intensity of LED 1 and LED2 as a function of wavelength. LED1 has a high intensity for a small wavelength band around 640 nm. LED2 has a high intensity for a small wavelength band around 870 nm. The light intensity of LED1 and LED2 as a function of the wavelength depends on the temperature of LED1 and LED2. For example, FIG. 4 depicts the light intensity of LED1 and LED2 as a function of wavelength at a desired temperature of 40°C. A shift of the temperature of LED1 and LED2 to a higher or a lower temperature causes a shift of the wavelength bands. Such a shift of the temperature causes, for example, that the maximum intensity of LED1 is not at 640 nm, but at a higher or lower wavelength. Such a shift of the temperature causes, for example, that the maximum intensity of LED2 is not at 870 nm, but at a higher or lower wavelength.

Optionally, the detector 212 is adapted to generate the detector signal based on sensor light source information. The sensor light source information comprises information about a spectral output of the at least one sensor light source, LED1 and LED2, at the desired temperature. The spectral output is, for example, the light intensity of LED1 and LED2 as a function of wavelength as depicted in FIG. 4.

Optionally, the detector 212 is adapted to generate the detector signal based on detector information. The detector information comprises information about a correlation between sensor light received by the detector 212 and the detector signal generated in response to the sensor light received by the detector 212 at the desired temperature. For example, the correlation is a non-linear correlation that depends on the spectrum and/or the intensity of the sensor light received by the detector 212.

FIG. 5 depicts a thermally conductive body 204 according to the first embodiment. The thermally conductive body 204 comprises a metal core printed circuit board, PCB, 500. The metal core PCB 500 has a core layer 502 comprising a metal, such as copper or aluminum. A dielectric layer 504 is arranged on the core layer 502. The detector 212 and the at least one sensor light source LED1, LED2, are arranged on the dielectric layer 504. Also the electric traces 209, for example, made from copper, are arranged on the dielectric layer 504 to electrically connect the detector 212 and the at least one sensor light source LED1, LED2. For example, the electric traces 209 connect the detector 212 and the at least one sensor light source LED1, LED2 to an electric power source via the electric connector 208. For example, one or more electric traces 209 couple the detector 212 to an output interface via the electric connector 208. The output interface generates an output signal based on the detector signal. For example, the output signal is representative of a preferred setting of the IPL apparatus 100 for the characteristic of the skin represented by the detector signal. The dielectric layer 504 provides an electrical insulation between the core layer 502 on one side, and on the opposite side the detector 212, the at least one light source LED1, LED2, and the electric traces 209. The core layer 502 has the thermally conductive material 206.

FIG. 6 depicts a thermally conductive body 204 according to a second embodiment. The second embodiment has, for example, the same features as the first embodiment, except for the following. In the second embodiment, the thermally conductive body 204 is a ceramic printed circuit board, PCB, 600. The ceramic PCB 600 has a ceramic layer 602. The ceramic layer 602 has the thermally conductive material 206. Because the ceramic layer 602 is electrically insulating, the detector 212, the at least one sensor light source LED1, LED2, and electric traces 209 are provided on the ceramic layer 602 without the need for the dielectric layer 504 in between. Because the detector 212 and the at least one sensor light source LED1, LED2, are in direct contact with the ceramic layer 602, heat is effectively transferred between the thermally conductive material 206, the detector 212 and the at least one sensor light source LED1, LED2. Optionally, the thermally conductive material 206 comprises aluminum oxide, or aluminum nitride, or boron nitride, or beryllium oxide, or silicon carbide. These materials have a very good thermal conductivity, and are electrically insulating.

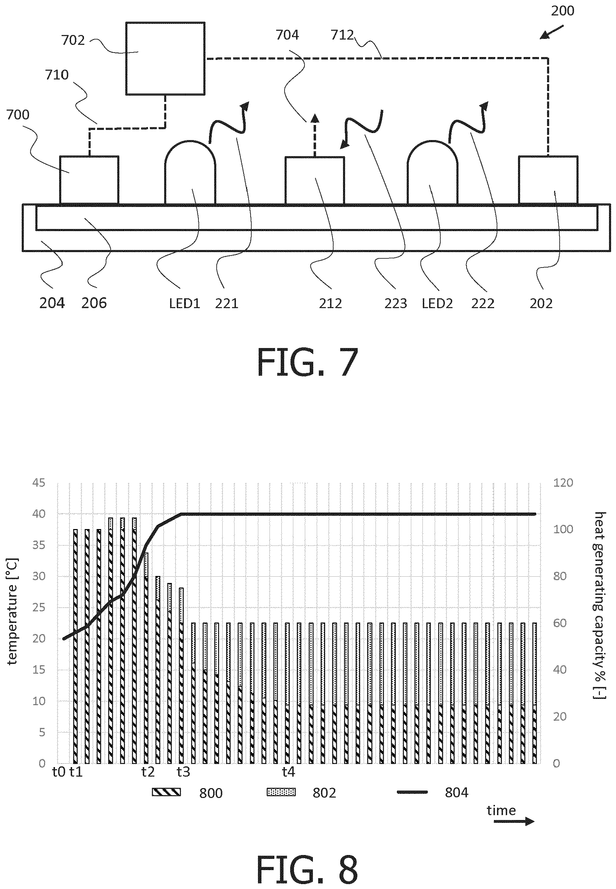

FIG. 7 depicts a sensor system 200 according to a third embodiment. The third embodiment has, for example, the same features as the first embodiment or as the second embodiment, except for the following. The heat control system comprises a temperature sensor 700. The temperature sensor 700 is thermally coupled to the thermally conductive material 206. The temperature sensor 700 is adapted to generate a temperature signal 710 representative of a temperature of the thermally conductive material 206. The heat control system is configured to control the temperature of the detection unit 210 based on the temperature signal 710.

The heat control system comprises a processing unit 702. The processing unit 702 receives the temperature signal 710. Based on the temperature signal 710, the processing unit 702 generates a heat control signal 712. Based on the heat control signal 712, the heating device 202 provides heat to the thermally conductive material 206. This way, the heat control system is able to control the temperature of the detection unit 210 at the desired temperature. For example, the processing unit 702 makes use of an on-off controller, a proportional (P) controller, a proportional and derivative (PD) controller or a proportional, derivative and integral (PID) controller to generate the heat control signal.

While at the desired temperature, LED1 is adapted to emit the first sensor light 221, and LED2 is adapted to emit the second sensor light 222. The first sensor light 221 and the second sensor light 222 are emitted to the skin. After interaction with the skin, at least a portion 223 of the sensor light reflected by the skin is received by the detector 212. The detector 212 is adapted to generate the detector signal 704 based on the at least a portion 223 of the sensor light reflected by the skin.

FIG. 8 depicts a temperature 804 of the detection unit 210 according to the third embodiment. Alternatively, the temperature 804 of the detection unit 210 as depicted in FIG. 8 is achieved according to the first embodiment or the second embodiment. The left y-axis indicates the temperature of the detection unit 210 in degrees Celsius. The x-axis indicates time.

At t=t0, the user switches on the IPL apparatus 100. FIG. 8 depicts that the temperature of the detection unit 210, for example the temperature of the thermally conductive material 206 as measured by the temperature sensor 700, is at an ambient temperature of 20°C at t=t0. The desired temperature of the detection unit 210 is 40°C. The temperature signal 710 is representative that the temperature of the detection unit 210 is less than the desired temperature. Based on the temperature signal 710, the heat control system controls the heating device 202 to provide heat 800 to the detection unit 210. The right y-axis indicates the amount of heat 800 generated by the heating device 202 relative to the maximum heat generating capacity of the heating device 202. The number 100 represents that the amount of heat 800 generated by the heating device 202 is 100% of the maximum heat generating capacity of the heating device 202. For example, the maximum heat generating capacity is 1W or 2W or 5W or 10W.

To achieve the desired temperature, the heating device 202 is controlled by the heat control system to heat at the maximum heat generating capacity from t=t1. The heating device 202 is controlled by the heat control system to heat at a lower capacity at t=t2 to prevent the temperature of the detection unit 210 to overshoot the desired temperature. At t=t3, the detection unit 210 is at the desired temperature of 40°C. The amount of heat needed to keep the detection unit 210 at the desired temperature is about 60% of the maximum heat generating capacity of the heating device 202.

Meanwhile, because of the use of the IPL apparatus 100, the treatment light source generates additional heat 802 that is transmitted to the detection unit 210. The additional heat 802 helps to increase the temperature 804 of the detection unit 210 from the ambient temperature to the desired temperature. The additional heat 802 increases over time. The amount of heat needed to keep the detection unit 210 at the desired temperature is partly provided by the additional heat 802. The remaining heat 800 is provided by the heat control system. As shown in FIG. 8, at t=t3, the additional heat 802 is a small portion of the total amount of heat. However, as the additional heat 802 increases due to use of the IPL apparatus 100, at t=t4, the additional heat 802 is a substantial portion of the total amount of heat. As a result, at t=t3, the heat control system controls the heating device 202 to provide a large amount of heat 800, i.e., about 65% of the maximum heat generating capacity. At t=t4, the heat control system controls the heating device 202 to provide a smaller amount of heat 800, i.e., about 25% of the maximum heat generating capacity.

From t=t4, the IPL apparatus 100 is in a thermal steady state. The total heat of 60% of the maximum heat generating capacity matches heat losses from the IPL apparatus 100 to the environment to keep the desired temperature at 40°C.

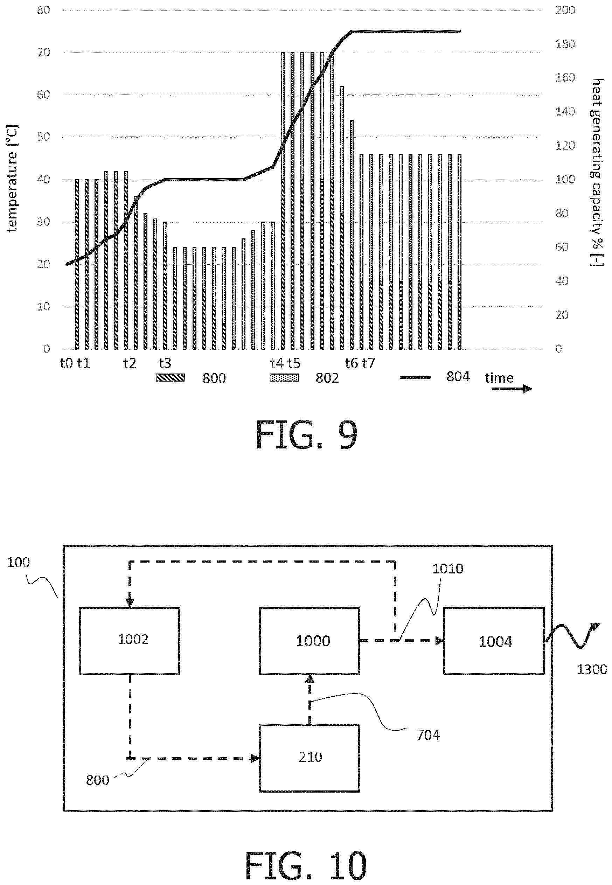

FIG. 9 depicts a temperature 804 of the detection unit 210 according to a fourth embodiment. For example, the temperature 804 is achieved using the sensor system 200 according to the first embodiment or the second embodiment or the third embodiment. The left y-axis indicates the temperature 804 of the detection unit 210 in degrees Celsius. The x-axis indicates time.

In the fourth embodiment, the heat control system is configured to control the temperature 804 of the detection unit 210 at a first desired temperature in case the temperature 804 of the thermally conductive material 206 is lower than a threshold temperature. The heat control system is configured to control the temperature 804 of the detection unit 210 at a second desired temperature in case the temperature 804 of the thermally conductive material 206 is higher than a threshold temperature. The second desired temperature is higher than the first desired temperature.

At t=t0, the user switches on the IPL apparatus 100. FIG. 9 depicts the temperature 804 of the detection unit 210, for example the temperature of the thermally conductive material 206 as measured by the temperature sensor 700, is at an ambient temperature of 20°C at t=t0. The first desired temperature of the detection unit 210 is 40°C. The temperature signal 710 is representative that the temperature 804 of the detection unit 210 is less than the first desired temperature. Based on the temperature signal 710, the heat control system controls the heating device 202 to provide heat 800 to the detection unit 210. The right y-axis indicates the amount of heat generated by the heating device 202 relative to the maximum heat generating capacity of the heating device 202. The number 100 represents that the amount of heat generated by the heating device 202 is 100% of the maximum heat generating capacity of the heating device 202. For example, the maximum heat generating capacity is 1W or 2W or 5W or 10W.

To achieve the first desired temperature, the heating device 202 is controlled by the heat control system to heat 800 at the maximum capacity till t=t2. The heating device 202 is controlled by the heat control system to heat at a lower capacity to prevent the temperature of the detection unit 210 to overshoot the first desired temperature. At t=t3, the detection unit 210 is at the first desired temperature of 40°C. The amount of heat needed to keep the detection unit 210 at the desired temperature is about 60% of the maximum heat generating capacity of the heating device 202.

Meanwhile, because of the use of the IPL apparatus 100, the treatment light source generates additional heat 802 that is transmitted to the detection unit 210. The additional heat 802 helps to increase the temperature 804 of the detection unit 210 from the ambient temperature to the first desired temperature. The additional heat 804 increases over time. The amount of heat needed to keep the detection unit 210 at the desired temperature is partly provided by the additional heat. The remaining heat is provided by the heat control system. As shown in FIG. 9, at t=t3, the additional heat 802 is a small portion of the total amount of heat. However, as the additional heat 802 increases due to use of the IPL apparatus 100, at t=t4, the additional heat 802 is more than the 60% of the maximum heat generating capacity. As a result, the temperature 804 of the detection unit 210 increases to above the first desired temperature. When the temperature 804 of the detection unit 210 exceeds the threshold temperature of 42 °C, at t=t5 the heat control system controls the heating device 202 to provide heat 800 at 100% of the heat generating capacity to bring the temperature 804 of the detection unit 210 to the second desired temperature of 75°C. At t=t6, the detection unit 210 reaches the second desired temperature. The heat control system controls the heating device 202 to provide heat 800 to maintain the detection unit 210 at the second desired temperature. From t=t7, the IPL apparatus 100 is in a thermal steady state The combination of the heat 800 provided by the heating device 202 and the additional heat 802 provided by the treatment light source matches with heat losses to the ambient environment to keep the detection unit 210 at the second desired temperature.

FIG. 10 depicts an IPL apparatus 100 according to a fifth embodiment. The fifth embodiment has, for example, the same features as the first embodiment, the second embodiment, the third embodiment or the fourth embodiment, except for the following.

In the fifth embodiment, the IPL apparatus 100 comprises a light control system 1000 adapted to provide a light control signal 1010 to the treatment light source 1004 to control operation of the treatment light source 1004. The heat control system 1002 is configured to control the temperature of the detection unit 210 based on the light control signal 1010 by providing heat 800 to the detection unit 210. The operation of the treatment light source 1004 comprises an operation time and/or a flashing frequency and/or an optical fluence of the treatment light source 1004.

Optionally, the light control system 1000 is adapted to receive the detector signal 704 from the detection unit 210. The light control system 1000 is adapted to control the treatment light source 1004 based on the detector signal 704. For example, the light control system 1000 is adapted to adjust a setting of the treatment light source 1004 based on the detector signal 704. The setting is, for example, an intensity or a flash frequency. For example, the light control system 1000 adjusts the setting of the treatment light source 1004 to provide improved treatment in view of characteristic of the skin as indicated by the detector signal 704. For example, the light control system 1000 is adapted to provide the light control signal 1010 to the treatment light source 1004 or not to provide the light control signal 1010 based on the detector signal 704. For example, in case the detector signal 704 is representative of characteristic of the skin indicating that the skin is not suitable to receive treatment light 1300, the light control system 1000 prevents the treatment light source 1004 to generate the treatment light 1300. For example, the skin is not suitable to receive the treatment light 1300 in case the skin has a mole or a scar or a tattoo.

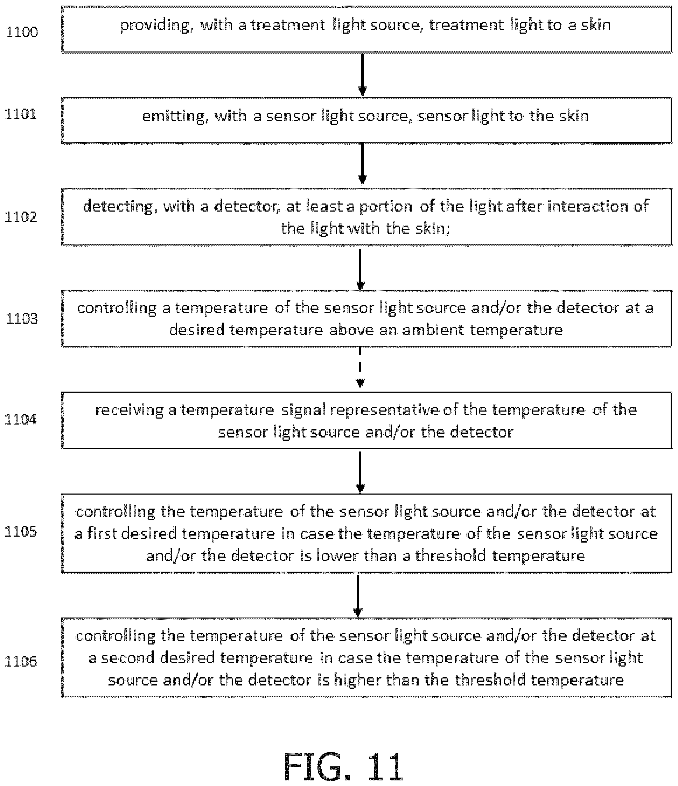

FIG. 11 depicts a method for operating the IPL apparatus 100 according to a sixth embodiment. The sixth embodiment has, for example, the same features as the first embodiment, the second embodiment, the third embodiment, the fourth embodiment, or the fifth embodiment, except for the following.

The method according to the sixth embodiment comprises providing, at 1100, with a treatment light source 1004, treatment light 1300 to a skin. The method comprises emitting, at 1101, with a sensor light source LED1, LED2, sensor light 221, 222, to the skin. The method comprises detecting, at 1102, with a detector 212, at least a portion 223 of the sensor light after interaction of the sensor light 221, 222 with the skin. The method comprises controlling, at 1103, a temperature 800 of the sensor light source LED1, LED2, and/or the detector 212 at a desired temperature above an ambient temperature.

Optionally, the method comprises receiving, at 1104, a temperature signal 710 representative of the temperature 804 of the sensor light source LED1, LED 2 and/or the detector 212. The method comprises controlling, at 1105, the temperature 804 of the sensor light source LED1, LED 2, and/or the detector 212 at a first desired temperature in case the temperature 804 of the sensor light source and/or the detector 212 is lower than a threshold temperature. The method comprises controlling, at 1106, the temperature 804 of the sensor light source LED1, LED2, and/or the detector 212 at a second desired temperature in case the temperature 804 of the sensor light source LED1, LED2, and/or the detector 212 is higher than the threshold temperature. The second desired temperature is higher than the first desired temperature.

As discussed above, the processing unit 702 is adapted to perform the data processing. The processing unit 702 can be implemented in numerous ways, with software and/or hardware, to perform the various functions required. The processing unit 702 may employ one or more microprocessors that may be programmed using software (e.g. microcode) to perform the required functions. The processing unit 702 may be implemented as a combination of dedicated hardware to perform some functions and one or more programmed microprocessors and associated circuitry to perform other functions.

The processing unit 702 may include circuitry. Examples of circuitry that may be employed include, but are not limited to, conventional microprocessors, application specific integrated circuits (ASICs), and field-programmable gate arrays (FPGAs). Thus, the processing unit 702 may be embodied as a digital and/or analog processing system.

In various implementations, the processing unit 702 may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Functions implemented by a processor may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems. The processing unit 702 may be configured to execute the computer program, causing the IPL apparatus to perform the method according to the sixth embodiment.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A light-based skin treatment device (100), comprising:
a treatment light source (1004) adapted to provide treatment light (1300) to a skin;
a sensor system (200) for measuring a characteristic of the skin,
wherein the sensor system (200) comprises a detection unit (210) having a detector (212) and at least one sensor light source (LED1, LED2),
wherein the at least one sensor light source (LED1, LED2) is adapted to emit sensor light (221, 222) to the skin,
wherein the detector (212) is adapted to generate a detector signal (704) in response to receiving at least a portion (223) of the sensor light after interaction of the sensor light (221, 222) with the skin;
wherein the sensor system (200) comprises a heat control system (1002) adapted to control a temperature (804) of the detection unit (210) at a desired temperature above an ambient temperature.

2. The light-based skin treatment apparatus (100) according to claim 1, wherein the heat control system (1002) comprises a heating device (202),
wherein the heating device (202) is adapted to provide heat (800) to the detection unit (210).

3. The light-based skin treatment apparatus (100) according to claim 1 or 2, wherein the detection unit (210) comprises a thermally conductive body (204),
wherein the thermally conductive body (204) comprises a thermally conductive material (206),
wherein the sensor light source (LED1, LED2) and the detector (212) are coupled to the thermally conductive body (204),
wherein the sensor light source (LED1, LED2), the detector (212), and the thermally conductive material (206) are thermally coupled to each other.

4. The light-based skin treatment apparatus (100) according to claim 3, wherein the heat control system (1002) comprises a heating device (202) thermally coupled to the thermally conductive material (206),
wherein the heating device (202) is adapted to provide heat (800) to the thermally conductive material (206).

5. The light-based skin treatment apparatus (100) according to claim 3 or 4, wherein the thermally conductive body (204) comprises one of a metal core printed circuit board (500) and a ceramic printed circuit board (600).

6. The light-based skin treatment apparatus (100) according to any one of claims 3-5, wherein the thermally conductive material (206) comprises aluminum oxide, or aluminum nitride, or boron nitride, or beryllium oxide, or silicon carbide.

7. The light-based skin treatment apparatus (100) according to any one of claims 3-6,
wherein the heat control system (1002) comprises a temperature sensor (700),
wherein the temperature sensor (700) is thermally coupled to the thermally conductive material (206),
wherein the temperature sensor (700) is adapted to generate a temperature signal (710) representative of a temperature (804) of the thermally conductive material (206),
wherein the heat control system (1002) is configured to control the temperature (804) of the detection unit (210) based on the temperature signal (710).

8. The light-based skin treatment apparatus (100) according to claim 7,
wherein the heat control system (1002) is configured to control the temperature (804) of the detection unit (210) at a first desired temperature in case the temperature (804) of the thermally conductive material (206) is lower than a threshold temperature,
wherein the heat control system (1002) is configured to control the temperature (804) of the detection unit (210) at a second desired temperature in case the temperature of the thermally conductive material (206) is higher than a threshold temperature,
wherein the second desired temperature is higher than the first desired temperature.

9. The light-based skin treatment apparatus (100) according to any one of the preceding claims, comprising
a light control system (1000) is adapted to provide a light control signal (1010) to the treatment light source (1004) to control operation of the treatment light source (1004),
wherein the heat control system (1002) is configured to control the temperature (804) of the detection unit (210) based on the light control signal (1010).

10. The light-based skin treatment apparatus (100) according to claim 9, wherein the operation of the treatment light source (1004) comprises an operation time and/or a flashing frequency and/or an optical fluence of the treatment light source (1004).

11. The light-based skin treatment apparatus (100) according to any one of the preceding claims, wherein the detector (212) is adapted to generate the detector signal (704) based on sensor light source information,
wherein the sensor light source information comprises information about a spectral output of the at least one sensor light source (LED1, LED2) at the desired temperature.

12. The light-based skin treatment apparatus (100) according to any one of the preceding claims, wherein the detector (212) is adapted to generate the detector signal (704) based on detector information,
wherein the detector information comprises information about a correlation between sensor light received by the detector (212) and the detector signal (704) generated in response to the sensor light received by the detector (212) at the desired temperature.

13. A sensor system (200) for measuring a characteristic of the skin,
wherein the sensor system (200) is adapted for use in the light-based skin treatment apparatus (100) according to any one of the preceding claims.

14. A method for operating a light-based skin treatment apparatus, the method comprising:
providing (1100), with a treatment light source (1004), treatment light (1300) to a skin;
emitting (1101), with a sensor light source (LED1, LED2), sensor light (221, 222) to the skin;
detecting (1102), with a detector (212), at least a portion (223) of the light after interaction of the light with the skin;
controlling (1103) a temperature (804) of the sensor light source (LED1, LED2) and/or the detector (212) at a desired temperature above an ambient temperature.

15. The method according to claim 14, comprising
receiving (1104) a temperature signal (710) representative of the temperature (804) of the sensor light source and/or the detector (212);
controlling (1105) the temperature of the sensor light source and/or the detector (212) at a first desired temperature in case the temperature of the sensor light source and/or the detector (212) is lower than a threshold temperature;
controlling (1106) the temperature of the sensor light source and/or the detector (212) at a second desired temperature in case the temperature of the sensor light source and/or the detector (212) is higher than the threshold temperature,
wherein the second desired temperature is higher than the first desired temperature.
